# EUROPÄISCHE PATENTSCHRIFT

(11) **EP 1 027 034 B1**
(45) Veröffentlichungstag und Bekanntmachung des Hinweises auf die Patenterteilung: **27.02.2002**
(21) Anmeldenummer: 98959791.9
(22) Anmeldetag: 16.10.1998
(51) Int. Cl.: A61K 9/00, A61K 41/00

(54) **MARKER FÜR EINE DARM-DIAGNOSTIK UND DARM-THERAPIE**
MARKER FOR INTESTINAL DIAGNOSIS AND INTESTINAL THERAPY
MARQUEUR POUR UN DIAGNOSTIC ET UN TRAITEMENT CONCERNANT L'INTESTIN

(30) Priorität: 17.10.1997 DE 19745890
(43) Veröffentlichungstag der Anmeldung: 16.08.2000
(73) Patentinhaber: INSTITUT FÜR PHYSIKALISCHE HOCHTECHNOLOGIE E.V., 07743 Jena (DE); Asclepion-Meditec AG, 07745 Jena (DE)
(72) Erfinder: ANDRÄ, Wilfried, D-07749 Jena (DE); WENDT, Michael, D-99423 Weimar (DE)
(74) Vertreter: Pfeiffer, Rolf-Gerd, Dipl.-Phys.
(86) Internationale Anmeldenummer: EP9806659
(87) Internationale Veröffentlichungsnummer: WO9920245

(56) Entgegenhaltungen:
- DE-A- 4 406 139
- DATABASE WPI Week 8126 Derwent Publications Ltd., London, GB; AN 81-46633d XP002092940 & JP 56 051411 A (KATO), 9. Mai 1981
- CHEMICAL ABSTRACTS, vol. 113, no. 12, 17. September 1990 Columbus, Ohio, US; abstract no. 103417, ITO, RITSUKO ET AL: "Pharmaceutical compositions containing magnetic substances for diseased area targeting" XP002092939 & JP 02 009813 A (DAINICHISEIKA COLOR AND CHEMICALS MFG. CO., LTD., JAPAN)

## Beschreibung

Die Erfindung betrifft einen Marker für eine Darm-Diagnostik und DarmTherapie. Der vorgeschlagene Marker ermöglicht die gleichzeitige Ortung seiner Position im Magen-Darm-Trakt als auch eine definierte Wirkstofffreisetzung in der gewünschten Behandlungszone.

Aus DE 196 00 744 Al ist die Möglichkeit des Einsatzes einer in Lösung befindlichen feinpulvrigen ferro- oder ferrimagnetischen EnergieAbsorber-Substanz zur lokalen Hyperthermie von Mamma-Karzinomen bekannt, wobei durch Anlegen eines magnetischen Wechselfeldes, infolge von magnetischen Verlusten, eine Erwärmung der AbsorberSubstanz bewirkt wird. Die zumindest über gewisse Zeiträume dort erreichte lokale Wirkung basiert auf dem Effekt der Tumorangiogenese.

Aus DE 195 32 676 C1 ist eine Anordnung zur Bestimmung der Position eines rein magnetischen Markers in einem Hohlraum innerhalb des Organismus eines Lebewesens, insbesondere im Magen-Darm-Trakt zu Diagnosezwecken, bekannt. Darüber hinaus ist in WO 97/09640 ein zugehöriges Verfahren, welches eine präzise Ortung eines solchen Markers betrifft, beschrieben.

Bekannt sind weiterhin organische Stoffe, die bei Erwärmung über Temperaturen im Bereich von ca. 30-60°C aus einem festen, z.B. gelartigen Zustand, in einen flüssigen Zustand übergehen. Beispiele dafür sind Mischungen aus Polylactid-Glycolid und Carrageenan. So wurde bspw. bei einer 2 Gew.% κ-Carrageenan-Lösung in Wasser eine Abnahme der Viskosität auf ca. 22% des Ausgangswertes festgestellt, wenn die Temperatur von 35°C auf 45°C erhöht wurde.
Durch Variation der Zusammensetzung solcher oder entsprechender Substanzen kann der Temperaturbereich des drastischen Viskositätsabfalls (Schmelzbereich) in ein vorgebbar gewünschtes Temperaturgebiet gelegt werden.

Weiterhin offenbart DATABASE WPI Week 8126 Derwent Publications Ltd. London, GB, AN 81-46633d XP002092940 & JP 56 051411 A (KATO), 9. Mai 1981 Mikrokapseln, die mit einem magnetischen Pulver versehen sind und einen medizinischen Wirkstoff beinhalten. Diese Mikrokapseln sollen vermittels magnetischer Gleichfelder an einen Behandlungszielort geführt und dort festgehalten werden. Die Auswahl des den medizinischen Wirkstoff umhüllenden Materials erfolgt nach dieser Schrift nach physiologischen Gesichtspunkten und nicht nach einem vorgebbaren Auflösungsverhalten. Weiterhin müssen die nach dieser Schrift zum Einsatz gelangenden Mikrokapseln offenbar sehr klein sein, damit sie ohne Gefahr des Verstopfens in Blutgefäße injiziert werden können. Das bedeutet, daß ihr Durchmesser kleiner als 5 um sein muß.

Aus DE 39 43 304 Al ist schließlich ein Verfahren zur lokalen Anwendung von Medikamenten bekannt, bei dem invasiv Elektroden in einen zu behandelnden Gewebebereich eingebracht werden, wobei durch Anlegung einer Gleichspannung die medikamentöse Wirkung auf den Elektrodenbereich begrenzt werden soll.

Der Erfindung liegt die Aufgabe zugrunde, einen Marker für eine Darm-Diagnostik und gleichzeitige Darm-Therapie anzugeben, dessen Position während seiner Wanderung durch den Magen-Darm-Trakt exakt bestimmbar ist und der in einer lokalen Behandlungszone eine kontrollierbare Wirkstofffreisetzung ermöglicht.

Die Aufgabe wird durch die kennzeichnenden Merkmale des Patentanspruchs 1 gelöst.

Das Wesen der Erfindung besteht darin, daß der Marker aus einem ferro- oder ferrimagnetischen Pulver, einer organischen Substanz mit einer temperatur im Bereich von 37-60°C zur Auflösung bringbar und einem Wirkstoff gebildet ist, wobei das ferro- oder ferrimagnetische Pulver in einer solchen Menge vorliegt, daß es innerhalb eines magnetischen Wechselfeldes geeigneter Feldstärke und Frequenz eine solche Erwärmung erfährt, die eine hinreichende Erniedrigung der Viskosität der organischen Substanz und damit ein Freisetzen des Wirkstoffes bewirkt. Beim Auflösen der organischen Substanz innerhalb der gewünschten Behandlungszone setzt gleichzeitig eine Verteilung des ferro- oder ferrimagnetischen Pulvers in größere Volumenbereiche ein, wodurch eine unzulässige weitere Temperaturerhöhung automatisch unterbunden wird.

Die Erfindung soll nachfolgend anhand von vier Ausführungsbeispielen und schematischen Zeichnungen näher erläutert werden. Es zeigen:

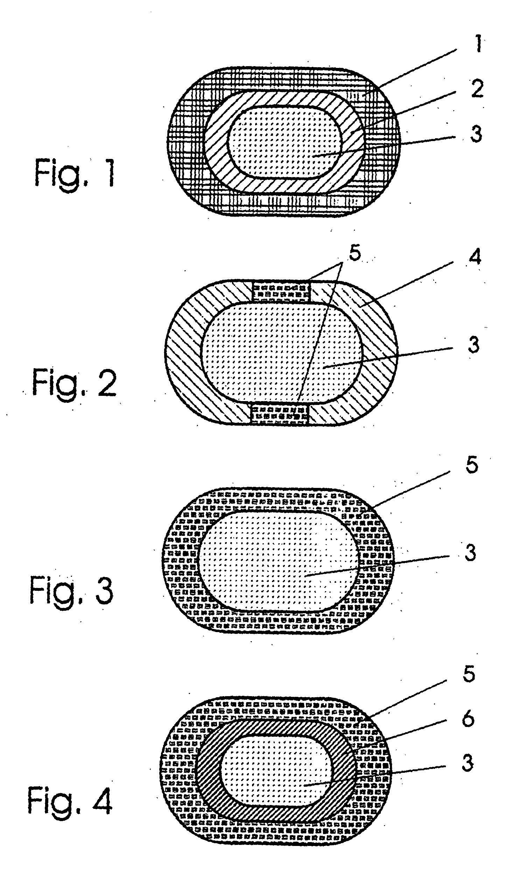

Fig. 1 bis 4 vier Ausbildungsmöglichkeiten eines erfindungsgemäßen Markers.

Fig. 1 zeigt schematisch einen ersten Marker im Längsschnitt, der gebildet ist durch eine den Wirkstoff 3 hüllenartig allseits umfassende organische Substanz 2, auf der direkt (oder indirekt über ein Bindemittel) eine Schicht aus einem ferro- oder ferrimagnetischen Pulver 1 aufgebracht ist. Bei dem ferro- oder ferrimagnetischen Pulver kann es sich insbesondere um nicht toxische Fe₃O₄-Teilchen oder γ-Fe₂O₃ handeln. Für die organische Substanz 2 finden solche Stoffe Verwendung, die in einem Temperaturbereich zwischen 37 - 60°C durch Erwärmung zur Auflösung bringbar sind. Im Beispiel wird hierfür eine Mischung aus 60 Masse% Carrageenan und 40 Masse% Resomer™ gewählt, die bspw. bei einer Temperatur von 45°C mit der Darmflüssigkeit in Lösung übergeht. Der Wirkstoff 3 kann in flüssiger oder fester Form vorliegen.

Figur 2 zeigt eine zweite Markerausbildung im Längsschnitt, bei der der Wirkstoff 3 hauptsächlich von einer im Magen-Darm-Trakt nicht' auflösbaren und nicht toxischen Hülle 4 umfaßt ist, wobei diese Hülle 4 in Teilbereichen unterbrochen ist, welche mit einer Mischung 5 aus einem Magnetitpulver 1 und der organischen, unter Wärmeeinfluß auflösbaren Substanz 2 verfüllt sind.

Figur 3 zeigt eine dritte Markerausbildung, bei der der Wirkstoff 3 allseitig von einer Mischung 5 aus einem Magnetitpulver 1 und einer organischen, unter Wärmeeinfluß auflösbaren Substanz 2 umhüllt ist.

Figur 4 zeigt schließlich eine vierte Markerausbildung, bei der der Wirkstoff 3 zunächst von einer im Magen-Darm-Trakt vollständig löslichen Hülle 6, z.B. aus Gelatine bestehend, umfaßt ist, welche ihrerseits mit einer Mischung 5 aus einem Magnetitpulver 1 und einer organischen, unter Wärmeeinfluß auflösbaren Substanz 2 umhüllt ist.

Weitere Markerausbildungen, denen das Prinzip des Einsatzes von ferro- oder ferrimagnetischen Pulvern und einer unter Wärmeeinfluß auflösbaren organischen Substanz zugrunde liegt, fallen unter den Rahmen der Erfindung.

Allen beschriebenen Ausführungsbeispielen ist gemein, daß sie eine energieabsorbierende Hülle oder Hüllenteilbereiche, entweder in Form einer Beschichtung mit ferro- oder ferrimagnetischen Pulvern oder in Form einer Mischung dieser Pulver mit einer unter Wärmeeinfluß auflösbaren Substanz, aufweisen. Der Wirkstoff wird mit genannter Umhüllung versehen, einem Patienten verabreicht und gelangt in dessen Magen-Darm-Trakt. Die Kontrolle der aktuellen Markerposition erfolgt bspw. nach WO97/09640. Ist die gewünschte Position im Behandlungsgebiet erreicht, wo der Wirkstoff freigesetzt werden soll, wird im Beispiel ein magnetisches Wechselfeld mit einer Amplitude von 24 kA/m und einer Frequenz von 300 kHz von außen an den Patienten über dem Markeraufenthaltsort angelegt. In diesem Fall ist eine Magnetitmasse von 10 mg im Marker ausreichend, um die unter Wärmeeinfluß auflösbare organische Substanz 2 innerhalb von 10 min von 37°C auf über 50°C zu erwärmen. Wenn die vorgebbare Schmelztemperatur in einem Gebiet, das zwischen 40 - 50°C liegen sollte, erreicht ist, wird die organische Substanz 2 erweicht, geht mit der Darmflüssigkeit in Lösung und die Magnetitteilchen, gleich, ob sie auf diese Hülle aufgebracht oder eingebracht sind, verteilen sich im Darm innerhalb kurzer Zeit auf ein Volumen, das mehr als 10mal größer ist, als das Ausgangsvolumen, welches sie im Marker eingenommen hatten. Dadurch wird auch bei weiterhin anliegendem magnetischen Wechselfeld die absorbierte Energie auf ein großes Gebiet verteilt, und wegen der damit verbundenen größeren Wärmekapazität steigt die Temperatur nicht weiter an.
Der wesentliche Vorteil bei Einsatz eines erfindungsgemäßen Markers besteht darin, daß eine nichtinvasive und von außen kontrollierbare Wirkstoffapplikation erfolgt, bei der der Patient geringstmöglichen Belastungen ausgesetzt ist.

## Patentansprüche

1. Marker für eine magnetische Darm-Diagnostik und Darm-Therapie, **dadurch gekennzeichnet, daß** der Marker aus einem ferro- oder ferrimagnetischen Pulver, einer organischen Substanz, die bei einer Temperatur im Bereich von 37-60°C zur Auflösung bringbar ist und einem Wirkstoff gebildet ist, wobei dem Marker eine kugelförmige oder eine medikamentenkapselähnliche äußere Gestalt gegeben ist und der ferro- oder ferrimagnetische Pulvermasseanteil im Marker entsprechend der Amplitude und Frequenz eines auf den Marker einwirkenden magnetischen Wechselfeldes derart festgelegt ist, daß die gewünschte Auflösungstemperatur der auflösbaren organischen Substanz erreichbar ist, wobei der ferro- oder ferrimagnetische Pulvermasseanteil im Marker in der Größenordnung von 10 mg festgelegt ist.

2. Marker nach Anspruch 1, **dadurch gekennzeichnet, daß** der Wirkstoff hüllenartig von einer Mischung aus einem ferro- oder ferrimagnetischen Pulver und der zur Auflösung bringbaren organischen Substanz umfaßt ist.

3. Marker nach Anspruch 1, **dadurch gekennzeichnet, daß** der Wirkstoff zunächst hüllenartig von der zur Auflösung bringbaren organischen Substanz umfaßt ist, auf welche das ferro- oder ferrimagnetische Pulver aufgebracht ist.

4. Marker nach Anspruch 1, **dadurch gekennzeichnet, daß** der Wirkstoff hüllenartig von der zur Auflösung bringbaren organischen Substanz umfaßt ist, in die in Teilbereichen ferro- oder ferrimagnetische Pulver eingebracht sind.

5. Marker nach Anspruch 1, **dadurch gekennzeichnet, daß** der Wirkstoff von einer unauflösbaren Hülle umfaßt ist, die in Teilbereichen unterbrochen ausgeführt ist und zumindest die unterbrochenen Bereiche mit einer zur Auflösung bringbaren organischen Substanz, in die ferro- oder ferrimagnetische Pulver ein- oder aufgebracht sind, verschlossen sind.

## Claims

1. Marker both for intestinal diagnosis and intestinal therapy, **characterized in that** said marker is constituted of a ferromagnetic and ferrimagnetic powder, respectively, an organic substance which is dissoluble at a temperature in a range of from 37-60°C and an active agent, wherein said marker is given an external spherical shape or a shape like a drug capsule, and wherein the part by weight of the ferromagnetic or ferrimagnetic powder in the marker is determined in accordance with the amplitude and frequency of the alternating magnetic field effecting the marker in a manner that the desired melting temperature of the organic substance to be dissolved is attainable, whereby the part by weight of the ferromagnetic or ferrimagnetic powder in the marker is determined to an order of size of 10 mg.

2. Marker as claimed in claim 1, **characterized in that** said active agent is cover-like enveloped by a mixture out of a ferromagnetic or ferrimagnetic powder and the organic substance is adapted for being dissolved.

3. Marker as claimed in claim 1, **characterized in that** said active agent at first is cover-like enveloped by the organic substance is adapted for being dissolved upon which then the ferromagnetic or ferrimagnetic powder is provided.

4. Marker as claimed in claim 1, **characterized in that** said active agent is cover-like enveloped by the organic substance is adapted for being dissolved in sections of which ferromagnetic or ferrimagnetic powder is inserted.

5. Marker as claimed in claim 1, **characterized in that** said active agent is enveloped by a non-dissolvable cover, sections of the same are intermittently embodied and wherein at least the intermittent sections are sealed by a organic substance is adapted for being dissolved into which and upon which, respectively, ferromagnetic or ferrimagnetic powder is provided.

## Revendications

1. Le marqueur utilisé pour le diagnostic et le traitement de pathologies du tractus gastro-intestinal est **caractérisé en ce que** le marqueur se compose d'une poudre ferro- ou ferrimagnétique, d'une substance organique qui, par des températures allant de 37°C à 60°C, se dissout, et d'un principe actif, que l'extérieur du marqueur est sphérique ou prend la forme de capsules de médicament, que le pourcentage de masse de la poudre ferro- ou ferrimagnétique dans le marqueur est définie, en fonction de l'amplitude et de la fréquence d'un champs magnétiques alternatif agissant sur le marqueur, de sorte que la température requise pour la dissolution de la substance organique soluble puisse être obtenue, le pourcentage de la masse de la poudre dans le marqueur étant fixé à 10 mg.

2. Le marqueur selon la revendication 1 est **caractérisé en ce que** le principe actif est enrobé d'un mélange d'une poudre ferro- ou ferrimagnétique et de la substance organique soluble.

3. Le marqueur selon la revendication 1 est **caractérisé en ce que** le principe actif est d'abord enrobé d'une substance organique sur laquelle est appliquée une couche de poudre ferro- ou ferrimagnétique.

4. Le marqueur selon la revendication 1 est **caractérisé en ce que** le principe actif est enrobé de la substance organique soluble subdivisée en segments, les interstices étant remplis de la poudre ferro- ou ferrimagnétique.

5. Le marqueur selon la revendication 1 est **caractérisé en ce que** le principe actif est enrobé d'une enveloppe indissoluble subdivisée en segments partiels séparés par des interstices qui sont remplis par des substances organiques solubles comprenant, à la surface ou à l'intérieur, une poudre ferro- ou ferrimagnétique.
